# EUROPEAN PATENT APPLICATION

(11) **EP 0 912 074 A1**
(43) Date of publication of application: **28.04.1999**
(21) Application number: 98119500.1
(22) Date of filing: 15.10.1998
(51) Int. Cl.: H04R 25/00, A61B 8/00

(54) **Determination of individual sensitivity to ultrasonic signals**

(30) Priority: 24.10.1997 US 957615
(71) Applicant: HEARING INNOVATIONS INCORPORATED, Tuscon, Arizona 85747 (US)
(72) Inventor: Green, Robert, S., Tucson, Arizona 85718 (US); Nunley, James, Scottsdale, Arizona 85258 (US); Brisken, Axel, F., Freemont, California 94539 (US)
(74) Representative: Ruschke, Hans Edvard, Dipl.-Ing.

(57) **Abstract**

A method and apparatus determining an individual's sensitivity to ultrasonic signals. As a result, devices which avail themselves of humans' ability to perceive ultrasonic signals, such as certain hearing aids and devices for echo location, tinnitus masking, and diagnosis and treatment of vestibular function disorders, can be effectively adjusted for the user.

## Description

### Background of the Invention

The present invention relates generally to methods and apparatus for determining an individual's sensitivity to ultrasonic signals.

It has been demonstrated that when normal hearing frequencies are shifted to the ultrasonic range and then transmitted by bone conduction or the like to the human sensory system, those frequencies are perceived. This has laid the foundation for some remarkable inventions. For example, an ultrasonic bone conduction hearing aid has been described.

The traditional hearing aid is an air-conduction amplifying system such that a microphone picks up air conduction sounds, amplifies them and presents them in the ear canals as an air conduction signal to the ear drum. These types of devices offer little or no benefit to the profoundly deaf, whose auditory systems are too severely damaged to make use of this amplification.

Bone conduction hearing aids have also been developed for users where the conventional hearing aid is not satisfactory. A bone conduction device is attached to the head of the user and the output from a microphone pick-up is amplified and fed into this device which causes bone vibration. These devices operate over a small dynamic range and are designed principally for individuals whose middle ears could not be surgically repaired. These bone conduction devices currently are not widely used.

The use of supersonic frequencies as a bone conducting hearing aid for normal hearing frequencies is a relatively new phenomenon. There has been mention of supersonic frequency detection in the literature but not for hearing aids. All known textbooks suggest that hearing stops at 20,000 Hertz.

In Lenhardt et al U.S. Patent No. 4,982,434, an invention is described whereby air conduction sounds in the conventional or audiometric range (which is a frequency range of about 100 to about 10,000 Hertz) are shifted into the supersonic range (which are frequencies above 20 kHz to about 108 kHz or higher) and then transmitted by bone conduction or the like to the human sensory system.

It is hypothesized that the ultrasonic bone conduction hearing aid is based on a system of hearing quite distinct from normal hearing based on air conduction. It utilizes bone conduction and parallels the primary hearing response of reptiles. In reptiles there is no air conduction hearing, but hearing is transmitted by bone conduction, mediated via the saccule which, in man, has been considered an organ responsible for balance and the determination of acceleration and movement.

Hearing in fish, amphibia and reptiles is mediated by vibratory frequencies that work through the vestibular system. In amphibia both bone and air conducted frequencies impinge on vestibular receptors. In reptiles air conduction hearing is nonexistent unless transduced via skin or bone to the vestibular saccule which is the primary hearing organ, as the cochlea does not exist. It has been postulated that during evolution, as mammals evolved from reptiles, therapsids or amphibia, so did the mammalian and avian cochlea which took over the role of the saccule as the primary hearing organ. The internal ear, or cochlea, is now the primary mammalian acoustic contact with the external environment. The saccule, although equipped with the neuro-corcial functional capacity to ascertain sound, became a back-up system of limited value, except for balance and motion detection. The awareness of the vestibular development role in evolutionary biology of hearing was lost as physiologists expanded on our understanding of the role of air conduction with clinical emphasis on the physiology and pathology of the cochlea.

The ultrasonic bone conduction hearing aid is believed to utilize direct bone transmission to the saccule and this enables hearing to be maintained via a system independent of air conduction and the inner ear although integrated with the air conduction system.

This discovery provided a new device for allowing the nerve deaf to hear. In addition, it provides an alternative source of informational transfer independent of sounds moving through air. The sound is transmitted directly to the bones of the skull, and utilizes frequencies that are perceived by the saccule and not by the inner ear. An advantage to utilization of the vestibule (saccule) as a hearing organ is that its response is transmitted via the vestibular nerve which can substitute for, or augment, communication in the acoustic nerve.

Apart from improving hearing in auditory nerve damaged users or hearing of those users suffering air conduction defects, Lenhardt et al also discloses that the supersonic bone conduction hearing invention will permit the perfection of echo location devices for the blind that should perform better than those currently under development. Further work in this area has also led to the discovery that the use of supersonic bone conduction can be used to mask or otherwise treat tinnitus, a condition best described as a ringing in the ears or head which does not emanate from an external source.

Absent in the prior art is any discussion of the effect of using different frequencies in the ultrasonic range for these and future inventions based on ultrasonic bone conduction. Does any frequency in the ultrasonic range work, or is one or more specific frequencies better than others? If specific frequencies are desirable, are there differences in individual perception of ultrasonic bone conduction stimulus so that the ultrasonic frequency must be carefully selected for the user?

We have determined that the individual's perception of ultrasonic signals transmitted by bone conduction does indeed vary from individual to individual. For some, they only detect the stimulus at a given frequency in the ultrasonic range. That is, for example, an individual may only perceive the stimulus in the 28 kHz range; introduce the stimulus to that person at, say 25 kHz, 32 kHz and the like, and that stimulus will not be perceived. For others, the stimulus will be perceived among a wide range of frequencies. And for others, it will be perceived among a wide range of frequencies, but it will be most effectively perceived in terms of ease of perception and comprehension in a particular frequency range. That is, for example, the individual may hear in the 25 kHz, 28 kHz and 32 kHz ranges, but the most effective frequency range for that individual is 28 kHz. As a result, the appropriate frequency for each user must be determined in order for any invention based on ultrasonic bone conduction to be effective.

Therefore, there exists in the art a need for an effective manner to determine an individual's sensitivity to ultrasonic stimuli. The present invention will enable the effective use of the existing and future discoveries based on ultrasonic bone conduction hearing by determining the correct ultrasonic frequency for each user.

### Summary of the Invention

The present invention overcomes the deficiencies in the prior art by providing a method and apparatus to determine an individual's sensitivity to ultrasonic frequency signals. In one embodiment of the invention, stimuli in the ultrasonic range are applied to the body at various frequencies and various sensation levels with the individual's sensitivity thereto recorded.

In particular, the present invention provides apparatus for determining an individual's sensitivity to ultrasonic signals, comprising means for generating signals in various ultrasonic frequency ranges and at various sensation levels, a means for applying said signals physically to a selected body part of said individual, and observing and recording the individual's sensitivity thereto.

Lenhardt et al U.S. Patent No. 4,932,434 discloses a hearing aid system which shifts sound signals from the auditory range to the ultrasonic frequency range (referred to in the patent as "supersonic" frequencies) and applies the ultrasonic signals to the human sensory system through bone conduction. The Lenhardt et al patent is incorporated herein by reference in its entirety.

### Brief Description of the Drawings

The present invention will become more fully understood from the detailed description given hereinbelow and the accompanying drawings, which are given for purposes of illustration only and are not limitative of the present invention, and wherein:
Figure 1 is a block diagram of the embodiment of the present invention;
Figure 2 is a diagram of the applicator affixed to an individual's head;
Figure 3 illustrates one individual's sensitivity to ultrasonic signals; and
Figure 4 illustrates another individual's sensitivity to ultrasonic signals.

### Detailed Description of the Preferred Embodiment

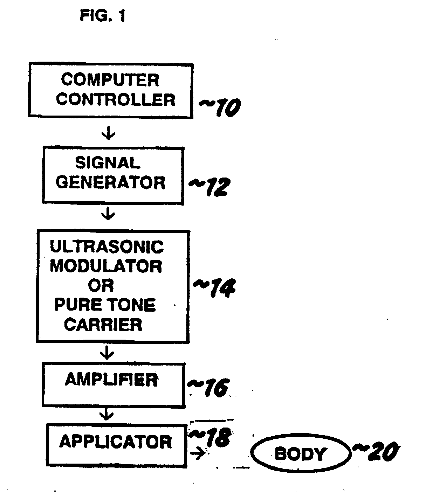

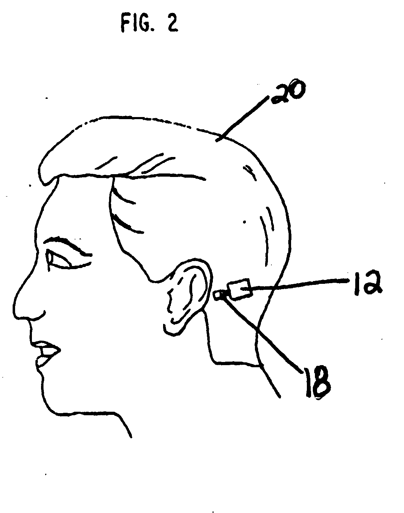

The present invention will now be described in detail with reference to Figures 1-4. A computer controller 10 controls the amplitude and frequency of a signal generator 12. The signal generator 12 develops signals which provide sensory stimuli in the auditory range when applied to an appropriate transducer (or applicator as referred to hereinafter). The signals developed by the signal generator 12 may include, but are not restricted to, pure tones, sine waves, square waves, white noise, tone bursts, warbles, or clicks. These various signals are generically referred to as signals hereinafter. The signals from the generator 12 are selected for maximum perception by the individual.

The signals from the generator 12 are inputted to an ultrasonic modulator or pure tone carrier 14 wherein they are transposed into the ultrasonic frequency range, which is above 20,000 Hertz and extends to approximately the 100,000 Hertz range. In particular, an ultrasonic carrier of 20,000 to 40,000 Hertz has been found to work well. As an alternative to the apparatus of Figure 1, the signals from the signal generator may originate in the ultrasonic frequency range in which case an ultrasonic modulator would not be needed. The ultrasonic signals are applied to an amplifier 16 for suitably increasing the amplitude of the signal.

The amplified signal from the amplifier 16 is applied to an applicator 18 for application to the body 20. Applicator 18 may be an electric/vibratory transducer, such as a piezo-electric driver, attached or applied to the skull for bone conduction, or it may be in the form of a speaker which creates physical vibrations in the air, which vibrations are transmitted in wave form through the air to physically impact a predetermined portion of the body which is responsive to physically applied vibrations for creating a perception in the brain. Additionally, the applicator 18 may be an electrode which directly applies an electromagnetic signal to a selected portion of the body.

Examples of suitable applicators are described in Lenhardt et al U.S. Patent No. 4,932,434 and in the co-pending United States patent application entitled "Device for Diagnosing and Treating Hearing Disorders," filed of even date herewith, which is incorporated herein by reference.

The signal is then altered, both in terms of frequency (within the ultrasonic range) and sensation level, which may be measured in force, decibels, newtons, sound pressure level or other method. In one embodiment of the invention, frequencies of 5,000 Hz intervals from 20,000 Hz to 45,000 Hz are introduced to the individual. For each such interval, the signal is presented at the lowest perceivable sensation level, and that frequency and sensation level is recorded. The individual gives some indication, for example, raising a hand when he senses the signal so that the data can be recorded.

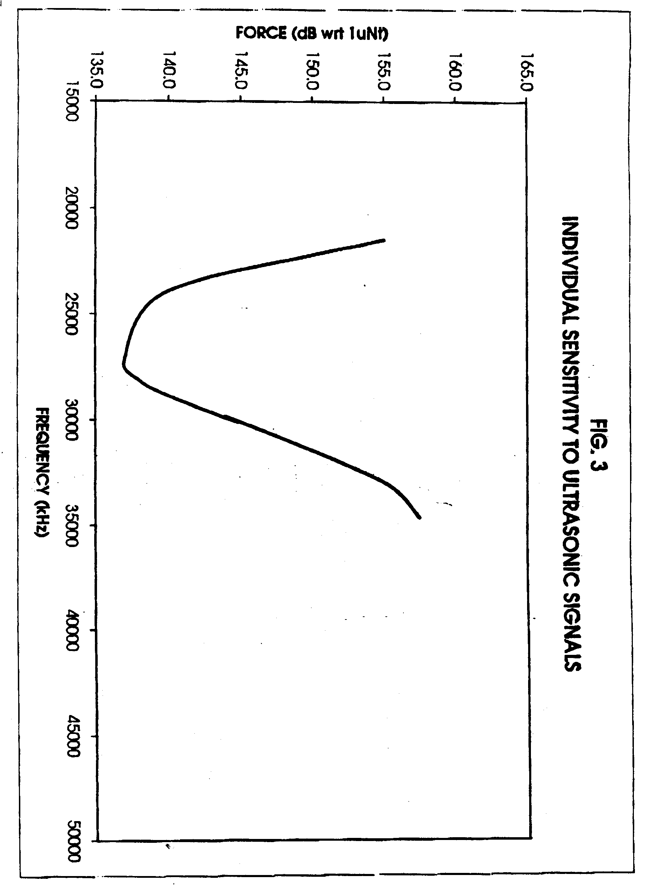

Figure 3 represents one form in which this data can be displayed. The chart plots the force in decibel micro newtons at which a particular individual first senses a signal at a particular frequency. The data provides a basis for selecting the most effective ultrasonic frequency, frequencies, or range of frequencies for use with the individual. For example, the frequency may be selected for which the decibel level for sensation is lowest.

Figure 3 indicates one individual's sensitivity to ultrasonic signals. The individual first senses a signal with a frequency of about 22 kHz at about 155 decibel micro newtons. The individual's most sensitive frequency is about 28 kHz at about 137 decibels micro newtons. Accordingly, ultrasonic bone conduction devices for that individual will advantageously use a frequency of about 28 kHz.

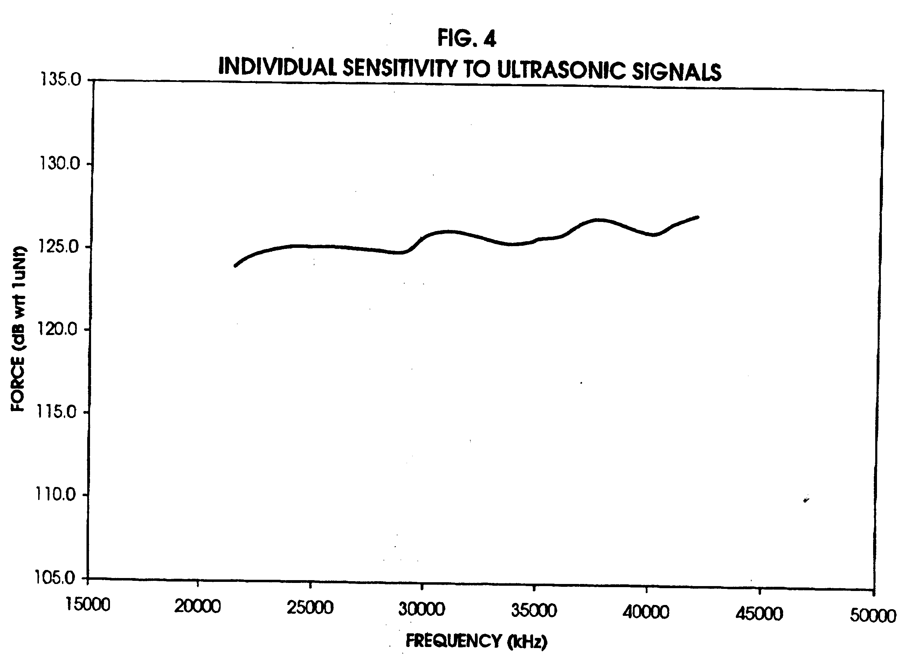

Figure 4 is a plot which indicates another individual's sensitivity to ultrasonic signals. The individual perceives the ultrasonic stimuli among a wide range of frequencies at generally the same force.

The invention can also be used to test a patient's vestibular function on the theory that if a patient cannot hear using the bone conduction device described herein, which we believe is mediated by the vestibular system, then there is a vestibular problem. The invention can also be used to treat vestibular function disorders say, for example, as a "vestibular masker" to lessen or alleviate motion sickness.

The invention having been described, it will be apparent to those skilled in the art that the same may be varied in many ways without departing from the spirit and scope of the invention. Any and all such modifications are intended to be covered by the following claims.

## Claims

1. A method for determining an individual's sensitivity to ultrasonic signals, comprising the steps of:
generating signals in the ultrasonic frequency range,
converting said signals to human sensory signals,
altering the frequency and sensitivity of said human sensory signals, and
recording the individual's perception of said altered signals.

2. The method of claim 1 in which said step of altering the sensitivity level comprises altering the decibels of the human sensory signals.

3. The method of claim 1 in which said step of altering the sensitivity level comprises altering the force of the human sensory signals.

4. The method of claim 1 in which said step of altering the sensitivity level comprises altering the sound pressure levels of the human sensory signals.

5. The method of claim 1 in which said step of converting said signals into human sensory signals comprises inputting said signals to an electric vibratory transducer and applying the transducer to the body of the individual to enable the individual to perceive the output of the transducer by bone conduction.

6. Apparatus for determining an individual's sensitivity to ultrasonic stimuli, comprising:
means for generating signals in the ultrasonic frequency range;
means for applying said signals physically to a selected body part of said individual; and
means for recording the individual's perception of the signals.

7. Apparatus according to claim 6 wherein said generating means comprises a signal generator which generates auditory signals in an auditory frequency range and an ultrasonic modulator for transposing said auditory signals to said ultrasonic frequency range.

8. Apparatus according to claim 7 wherein said auditory signals are pure tone signals.

9. Apparatus according to claim 7 wherein said auditory signals are white noise signals.

10. Apparatus according to claim 7 wherein said auditory signals are sine wave signals.

11. Apparatus according to claim 7 wherein said auditory signals are square wave signals.

12. Apparatus according to claim 7 wherein said auditory signals are tone bursts.

13. Apparatus according to claim 7 wherein said auditory signals are warble.

14. Apparatus according to claim 6 wherein said generating means comprises a signals generator which generates signals in said ultrasonic frequency range.

15. Apparatus according to claim 14 wherein said auditory signals are pure tone signals.

16. Apparatus according to claim 14 wherein said auditory signals are white noise signals.

17. Apparatus according to claim 14 wherein said auditory signals are sine wave signals.

18. Apparatus according to claim 14 wherein said auditory signals are square wave signals.

19. Apparatus according to claim 14 wherein said auditory signals are tone bursts.

20. Apparatus according to claim 14 wherein said auditory signals are warbble.

21. Apparatus according to claim 6 wherein said applying means comprises an electric/vibratory transducer.

22. Apparatus according to claim 1 wherein said generating means includes means for altering the frequency and sensation level of signals.

23. Apparatus according to claim 22 wherein said recording means comprises manually recorded plot of the frequency and sensation level of said signals.
